# EUROPÄISCHE PATENTSCHRIFT

(11) **EP 0 766 586 B1**
(45) Veröffentlichungstag und Bekanntmachung des Hinweises auf die Patenterteilung: **26.08.1998**
(21) Anmeldenummer: 95923306.5
(22) Anmeldetag: 12.06.1995
(51) Int. Cl.: B01D 19/04

(54) **WÄSSRIGE FETTALKOHOLDISPERSIONEN**
AQUEOUS FATTY ALCOHOL DISPERSIONS
DISPERSIONS AQUEUSES D'ALCOOLS GRAS

(30) Priorität: 20.06.1994 DE 4421270
(43) Veröffentlichungstag der Anmeldung: 09.04.1997
(73) Patentinhaber: Henkel Kommanditgesellschaft auf Aktien, 40191 Düsseldorf (DE)
(72) Erfinder: WOLLENWEBER, Horst-Werner, D-40597 Düsseldorf (DE); HÖFER, Rainer, D-41464 Neuss (DE); SCHULTE, Heiz-Günther, D-45481 Mühlheim (DE)
(86) Internationale Anmeldenummer: EP9502260
(87) Internationale Veröffentlichungsnummer: WO9535143

(56) Entgegenhaltungen:
- EP-A- 0 230 977
- US-A- 4 009 119
- US-A- 4 340 500
- DATABASE WPI Section Ch, Week 8323 Derwent Publications Ltd., London, GB; Class A97, AN 83-55096 & JP,A,58 072 000 (DAIICHI KOGYO SEIYAKU) , 28.April 1983

## Beschreibung

Die Erfindung betrifft wäßrige Fettalkohol-Dispersionen sowie ihre Verwendung als Antischaummittel, vorzugsweise in der Papierindustrie, im Abwasserbereich, in der Bauindustrie und bei der Herstellung von Wasserlacken sowie ihre Verwendung als Additiv für dispersionsgebundene Putze.

Für eine Vielzahl technischer Prozesse stellt die mit der Anwesenheit bzw. mit dem Einsatz von grenzflächenaktiven Substanzen verbundene Schaumentwicklung ein ernsthaftes Problem dar. So tritt beispielsweise bei der Herstellung von Farben und Lacken beim Anreiben des Lacks durch Einrühren von Luft verstärkte Schaumbildung auf. Aufgrund dieser Schaumbildung gestaltet sich die Herstellung von Farben und Lacken langwieriger, da die Anlagen nur mit einem Bruchteil des zur Verfügung stehenden Volumens gefüllt und betrieben werden können. Gleichzeitig kann eine solche Schaumentwicklung aber auch auftreten, wenn der Anwender Lacke bzw. Farben auf die Untergründe auftragen will. Dabei können Luftbläschen entstehen, die nicht nur optische Oberflächenstörungen darstellen, sondern auch Schwachstellen des getrockneten Films sind, da die Bläschen leicht durch Stoßeinwirkung platzen können.

Auch in der Papierfabrikation können aufgrund des im Wasserkreislauf eingetragenen Luftgehaltes Schäume gebildet werden, die zu Störungen führen. Beispielsweise können Schaumflecken auf dem Papier auftreten, wenn Schaum bei der Blattbildung auf die Papierbahn gelangt. Durch die zunehmend schneller laufenden Maschinen steigt insgesamt die Gefahr der Untermischung von Luft in die Fasersuspension, was zu einer Störung des Entwässerungsvorgangs des Papierstoffs auf der Papiermaschine und letztendlich zu porösen Strukturen des Papierblattes führen kann.

Diese prinzipiell bekannten Nachteile werden durch die neuen Papiermaschinen mit den geschlosseneren Wasserkreisläufen noch verstärkt, da sich schaumbildende und schaumstabilisierende Stoffe in den geschlossenen Systemen anreichern. Wie die obigen Ausführungen zeigen, besteht in der Technik ein großer Bedarf sowohl an Entschäumern, die in der Lage sein sollen, entstandenen Schaum zu verringern, als auch an Schauminhibitoren, die das Entstehen von Schaum unterdrücken sollen. Des weiteren sollen Antischaummittel auch in flüssigen Systemen gelöste Luftbläschen an die Systemoberfläche treiben, ein Vorgang, der als Entgasen oder auch als Entlüften, beispielsweise bei Lacken eine große Rolle spielt. Antischaummittel sind daher im Sinne der Erfindung Mittel, die sowohl entstehenden Schaum verringern und präventiv die Schaumbildung inhibieren als auch Luftbläschen austreiben sollen. Von diesen Antischaummitteln wird erwartet, daß sie in geringen Zusatzmengen kurzfristig aber auch langanhaltend wirksam sind.

In der Bauindustrie werden zur Lösung von Schaumproblemen Entschäumer gesucht, die wirksam sind, keine Oberflächenstörungen verursachen, keine unerwünschte Wasserempfindlichkeit der Oberflächen bewirken und z.B. in Gipsplatten die Adhäsion nicht stören.

Bei dispersionsgebundenen Putzen, wie bei Kunstharz- und Silikat-gebundenen Putzen, wird für die Applikation ein möglichst langer Verarbeitungszeitraum gewünscht, um gute Verreibbarkeit und gewünschte Putzstrukturen erhalten zu können. Die rezepturbedingten hohen Anteile an Zuschlagstoffen sowie die Applikation auf meist saugfähigen Untergründen bewirken eine rasche Abgabe des Wasseranteils, wodurch die Verarbeitungszeit ("offene Zeit") stark eingeschränkt wird. Sobald der Verfestigungsprozeß, also die Trocknung, einsetzt, ist die Möglichkeit zur Strukturierung der Oberfläche beendet. Vor allem bei höheren Umgebungstemperaturen führt rasche Wasserabgabe zu einer kritischen Einschränkung des Verarbeitungszeitraumes der dispersionsgebundenen Putze. Es besteht in der Technik daher ein großer Bedarf an Additiven, die in der Lage sind, die Verarbeitungszeit der dispersionsgebundenen Putze zu verlängern.

Auf beiden Anwendungsgebieten kommen wäßrige Fettalkoholdispersionen zum Einsatz. So beschreibt beispielsweise die deutsche Offenlegungsschrift DE-A-36 01 929 Entschäumer auf Basis von Öl-in-Wasser-Emulsionen, bei denen die Öl-Phase der Emulsion einen C₁₂-C₂₆-Alkohol, einen Fettsäureester und einen Kohlenwasserstoff enthält und als Emulgator grenzflächenaktive Stoffe eingesetzt werden, wie beispielsweise Natrium- oder Ammoniumsalze höherer Fettsäuren, oxalkylierte Alkylphenole, oxethylierte ungesättigte Öle oder sulfatierte Oxethylierungsprodukte von Nonylphenol oder Octylphenol.

In der deutschen Offenlegunsschrift DE-A-30 39 393 wird eine wäßrige Antischaummittelzusammensetzung beschrieben, die Wasser, mindestens einen höheren aliphatischen Alkohol, mindestens eine feste Fettsäure, mindestens eine Seife einer festen Fettsäure und oberflächenaktive Mittel enthält. Diese oberflächenaktiven Mittel können anionische und/oder nichtionische Tenside sein. Als nichtionische Tenside werden Ethylenoxid-Kondensationsprodukte erwähnt, wobei mindestens eine Endgruppe davon durch Kondensation mit einem Alkohol, Alkylphenol oder einer langkettigen Fettsäure terminiert ist.

Bei den in den deutschen Offenlegungsschriften DE-A-36 01 929 und DE-A-30 39 393 enthaltenen höheren aliphatischen Alkoholen handelt es sich um Synthesealkohole, sogenannte Alfol-Alkohole, die im Verhältnis zu natürlichen Fettalkoholen unproblematischer zu dispergieren sind. Des weiteren werden in beiden Offenlegungsschriften als nichtionische Tenside ethoxylierte Alkylphenole favorisiert. Alkylphenolethoxylate sind toxikologisch nicht unbedenklich und zudem nur schlecht biologisch abbaubar, so daß auf diese Verbindungsklasse verzichtet werden sollte. Insgesamt gesehen sind die in den beiden deutschen Offenlegungsschriften vorgeschlagenen anionischen und/oder nichtionischen grenzflächenaktiven Verbindungen zwar geeignet, synthetische höhere Alkohole zu stabilisieren, aber bei den natürlichen Fettalkoholen versagen sie, vor allem, wenn höhere Gehalte an natürlichem Fettalkohol über etwa 20 Gew.-% in den Fettalkoholdispersionen gewünscht werden. So treten insbesondere bei diesen Gehalten an natürlichen Fettalkoholen bei langer Lagerung oder bei Lagerung bei Temperaturen unter 15°C häufig schon nach einem Tag Gelbildung oder Aufrahmungen auf.

Aufgabe der vorliegenden Erfindung war es daher, wäßrige Fettalkoholdispersionen zur Verfügung zu stellen, die mit grenzflächenaktiven Verbindungen stabilisiert sind, die aromatenfrei und biologisch abbaubar sind. Zudem sollten die Dispersionen mit den schwierig zu dispergierenden natürlichen Fettalkoholen lagerstabil sein. Diese Lagerstabilität sollte vor allem auch bei Temperaturen unter Raumtemperatur über einen längeren Zeitraum als bisher gegeben sein. Darüber hinaus sollten die Fettalkoholdispersionen leicht dosierbar und über einen breiten Temperaturbereich anwendbar und lagerstabil sein. Für ihre Anwendung als Antischaummittel galt es, wäßrige Fettalkoholdispersionen zur Verfügung zu stellen, die zudem eine große Wirksamkeit zeigen. Bei der Anwendung in den dispersionsgebundenen Putzen sollten die wäßrigen Fettalkoholdispersionen in der Lage sein, die Verarbeitungszeit zu verlängern.

Überraschenderweise können wäßrige Fettalkoholdispersionen, die als nichtionische grenzflächenaktive Verbindungen Umsetzungsprodukte von Ethylenoxid mit 1,2-Alkandiolen mit 6 bis 18 C-Atomen enthalten, die Anforderungen erfüllen.

Sinngemäß betrifft die vorliegende Erfindung wäßrige Fettalkoholdispersionen, enthaltend
- C₁₀-C₂₈-Fettalkohole in der Ölphase sowie
- anionische grenzflächenaktive Verbindungen und
- nichtionische grenzflächenaktive Verbindungen und
- Wasser,
dadurch gekennzeichnet, daß die nichtionischen grenzflächenaktiven Verbindungen Umsetzungsprodukte von Ethylenoxid mit 1,2-Alkandiolen mit 6 bis 18 C-Atomen sind.

Bevorzugt werden als nichtionische grenzflächenaktive Verbindungen solche der allgemeinen Formel (I) 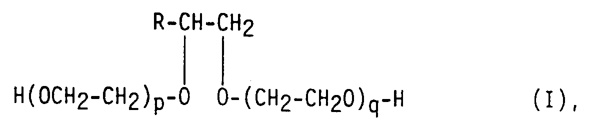 in der R einen über Kohlenstoff gebundenen gesättigten oder ungesättigten Rest mit 6 bis 18 C-Atomen und p und q eine Zahl von 0 bis 50 bedeutet, wobei die Summe von p und q im Bereich von 5 bis 50 liegt.

Verbindungen der allgemeinen Formel (I) sind als Umsetzungsprodukte von Ethylenoxid mit aliphatischen vicinalen endständigen Alkandiolen bereits bekannt. In der deutschen Offenlegungsschrift DE-A- 40 06 391 werden Tensidmischungen als Emulgatoren dür die Emulsionspolymerisation beschrieben, die neben ethoxylierten Alkoholen auch Umsetzungsprodukte von Ethylenoxid mit 1,2-Alkandiolen enthalten. Bei der Emulsionspolymerisation werden derartige Tensidmischungen zunächst für die Emulgierung der flüssigen Monomere und später für deren Polymerisate benötigt. Bei der vorliegenden Erfindung werden die nichtionischen grenzflächenaktiven Verbindungen mit anionischen grenzflächenaktiven Verbindungen abgemischt, um die in Wasser unlöslichen Fettalkohole dispergieren zu können. Des weiteren ist in der DE-A-40 06 391 kein Hinweis auf verbesserte Lagerstabilität zu finden.

Die Herstellung der Umsetzungsprodukte von Ethylenoxid mit 1,2 Alkandiolen kann wie in der DE-A-40 06 391 beschrieben erfolgen. Weitere Aufschlüsse gibt die deutsche Patentschrift DE-B-11 90 927, die die Umsetzung von 1,2-Diolen mit 8 bis 26 C-Atomen mit Ethylenoxid beschreibt, sowie die deutsche Offenlegungsschrift DE-A-29 00 030, die ein Verfahren zur Ringöffnung innenständiger oder endständiger Olefinepoxide, die 6 bis 18 C-Atome enthalten, mit mehrfunktionellen Alkoholen wie Ethylenglykol und nachfolgender Ethoxylierung der Reaktionsprodukte, offenbart.

Im Rahmen der Erfindung werden Umsetzungsprodukte von Ethylenoxid mit gesättigten oder ungesättigten 1,2-Alkandiolen mit 6 bis 18 C-Atomen als Verbindungen der allgemeinen Formel (I) eingesetzt. Bevorzugt im Rahmen der Erfindung werden als Verbindungen der allgemeinen Formel (I) Anlagerungsprodukte von Ethylenoxid an lineare 1,2-Alkandiole mit gerader oder ungerader Kohlenstoffzahl oder deren Mischungen. Ganz besonders geeignet sind ethoxylierte 1,2-Alkandiole mit 12 und/oder 14 C-Atomen. Die Herstellung solcher ethoxylierten 1,2-Alkandiole erfolgt in bekannter Weise aus Olefinen und Olefingemischen mit endständigen Doppelbindungen durch Epoxidation und anschließender katalytischer Ringöffnung der resultierenden endständigen Epoxyalkane mit Ethylenglykol. Die Ringöffnungen der 1,2-Epoxyalkane mit Ethylenglykol kann sauer oder basisch katalysiert werden. Bei der sauren Katalyse entstehen etwa zur Hälfte 1-Hydroxy-2-(2-hydroxyethoxy)-2-alkane, die zwei primäre Hydroxylgruppen aufweisen und zur anderen Hälfte entstehen dabei 1-(2-Hydroxyethoxy-)-2-hydroxyalkane, die eine primäre und eine sekundäre Hydroxylgruppe aufweisen. Bei den Verbindungen, die zwei primäre Hydroxylgruppen aufweisen, können diese in praktisch gleicher Weise mit Ethylenoxid weiterreagieren. Die Verbindungen, die eine primäre und eine sekundäre Hydroxylgruppe aufweisen, zeigen eine bevorzugte Weiterreaktion mit Ethylenoxid an der primären Hydroxylgruppe. Weiterhin ist es möglich, die Ringöffnung der 1,2-Epoxyalkane mit einem Überschuß an Ethylenglykol basisch zu katalysieren. Dabei entstehen im wesentlichen 1-(2-Hydroxyethoxy-)-2-hydroxyalkane. Im Rahmen der Erfindung werden Verbindungen der allgemeinen Formel (I) bevorzugt, die durch sauer katalysierte Ringöffnung von endständigen Epoxyalkanen mit Ethylenglykol und anschließender Epoxydierung hergestellt worden sind.

Durch Ethoxylierung werden die 1,2-Alkandiole in die Verbindungen der allgemeinen Formel (I) überführt. Die Ethoxylierung kann nach aus dem Stand der Technik bekannten Verfahren erfolgen, die je nach Wahl der Alkoxylierungskatalysatoren zu Produkten mit enger oder mit breiter Homologenverteilung führen. Die Wahl des Alkoxylierungskatalysators beeinflußt dabei die Breite des Spektrums von Anlagerungsprodukten, die sogenannte Homologenverteilung, des Ethylenoxids an die 1,2-Alkandiole. So werden in Gegenwart der katalytisch wirkenden Alkalimetallalkoholate wie Natriummethylat Anlagerungsprodukte mit breiter Homologenverteilung erhalten, während beispielsweise in Gegenwart von Hydrotalcit als Katalysator stark eingeengte Homologenverteilung erfolgt.

Im Rahmen der Erfindung werden Verbindungen der allgemeinen Formel (I) eingesetzt, die durch Umsetzung von aliphatischen 1,2-Alkandiolen mit bis zu 49 mol Ethylenoxid pro mol Alkandiol erhalten werden, wobei die beiden Hydroxylgruppen der 1,2-Alkandiole durch unterschiedliche Mengen an Ethylenoxid angelagert haben können. Damit können p und q unabhängig voneinander eine Zahl zwischen 0 bis 50 bedeuten, wobei jedoch gegeben sein muß, daß die Summe von p und q im Bereich von 5 bis 50, vorzugsweise im Bereich von 5 bis 30. Bei den Indices p und q ist zu beachten, daß dies nicht den gesamten Ethoxylierungsgrad darstellt, da das zur Ringöffnung eingesetzte Ethylenglykol mit zu berücksichtigen ist.

Die Fettalkohole, die in den wäßrigen Fettalkoholdispersionen enthalten sind, weisen vorzugsweise Schmelzpunkte über 40 °C, insbesondere über 50 °C auf. Bei den Fettalkoholen kann es sich um synthetische und/oder natürliche Fettalkohole handeln. Bevorzugt werden natürliche Fettalkohole, die gesättigt und gerad- und/oder verzweigtkettig sind, und 16 bis 28 C-Atome haben. Beispiele für die geradzahligen Fettalkohole sind Palmityl-, Stearyl-, Arachidyl-, Behenyl-, Lignoceryl- und Cerylalkohol sowie Octacosanol-1.

Die geradzahligen Fettalkohole können wie in der Fettchemie üblich auch in Form technischer Schnitte eingesetzt werden, wie sie beispielsweise gegebenenfalls nach Absättigung der ungesättigten Anteile aus erucasäure-reichem Rüböl, Erdnußöl, Rizinusöl, Rindertalg oder Fischöl durch Hydrierung erhalten werden. Abgesehen von diesen natürlichen Fettalkoholen sind im Sinne der Erfindung auch gesättigte, geradkettige synthetische Fettalkohole mit 16 bis 28 C-Atomen geeignet, die nach dem Ziegler-Verfahren durch Oxidation von Aluminiumalkylen und anschließender Hydrolyse erhältlich sind. Auf diese Weise werden Mischungen von geradzahligen, geradkettigen Fettalkoholen (Alfole) erhalten. Auch synthetische Fettalkohole, die durch die Oxosynthese durch Umsetzung von Olefin mit Kohlenmonoxid/Wasserstoff erhalten werden, wie ungeradzahlige Alkohole, sind im Sinne der Erfindung geeignet.

Besonders bevorzugt werden gesättigte, unverzweigte Fettalkohole mit 16 bis 22 C-Atomen natürlichen Ursprungs und insbesondere Mischungen von solchen, die zwischen 20 bis 70 Gew.-% Stearylalkohol enthalten.

Die erfindungsgemäßen wäßrigen Fettalkoholdispersionen enthalten die Fettalkohole in Mengen von 5 bis 40 Gew.-%, vorzugsweise 15 bis 35 Gew.-% - bezogen auf wäßrige Fettalkoholdispersionen.

Die erfindungsgemäßen wäßrigen Fettalkoholdispersionen können zusätzlich 0,5 bis 10 Gew.-%, an Stelle der entsprechenden Mengen des Fettalkohols, natürliche und/oder synthetische Wachse, aliphatische C₁₄₋₂₈-Fettsäuren und/oder Alkalimetall- oder Aminseifen von aliphatischen C₁₄₋₂₈-Fettsäuren enthalten. Natürliche Wachse sind im Sinne der Erfindung sowohl solche, die bei der Verarbeitung von Erdöl, Bitumen und/oder Fossilien erhältlich sind, beispielsweise die Paraffinwachse und Montanwachse als auch die natürlichen Ester, Ketone oder Amide von natürlichen C₁₆-C₃₂-Fettsäuren oder von natürlichen C₁₆-C₃₂-Fettalkoholen wie Stearylstearat, Stearylbehenat, Behenylbehenat, Stearon, Glycerintristearat, Glycerintribehenat, Pentaerythrittetrastearat, gehärtetes Ricinusöl, Ethylenbisstearamid, Erucasäureamid, Carnaubawachs, Bienenwachs usw. Besonders bevorzugt im Sinne der Erfindung werden die natürlichen Wachse und insbesondere die an und für sich üblichen Paraffinwachse mit Schmelzpunkten nach DIN 51570 über 50°C. Sollten Fettsäurekomponente, Fettsäureseifenkomponente und/oder Wachse in entsprechendem, den Fettalkohol ersetzenden Anteil, zugegeben sein, so beträgt die vorhandene Mindestmenge des Fettalkohols in der Zusammensetzung mindestens 5 Gew. -%, vorzugsweise mindestens 10 Gew. -%.

Die erfindungsgemäßen wäßrigen Fettalkoholdispersionen erhalten des weiteren anionische grenzflächenaktive Verbindungen ausgewählt aus der Gruppe Alkoholsulfate, ethoxylierte Alkoholsulfate, Fettalkoholethersulfosuccinate und Fettalkoholetherphosphate. Besonders bevorzugt als anionische grenzflächenaktive Verbindungen werden C₈₋₁₈-Fettalkoholethersulfate mit 6 bis 100 mol Ethylenoxid, vorzugsweise mit 20 bis 60 Mol Ethylenoxid. Geeignete Beispiele hierfür sind Natriumlaurylethersulfat ethoxyliert mit 30 Mol Ethylenoxid und Natriumlaurylethersulfat ethoxyliert mit 50 Mol Ethylenoxid.

Die erfindungsgemäßen wäßrigen Fettalkoholdispersionen enthalten die nichtionischen grenzflächenaktiven Verbindungen in Mengen von 0,5 bis 5 Gew.-% und die anionischen grenzflächenaktiven Verbindungen auch in Mengen von 0,5 bis 5 Gew.-%.

Somit ergeben sich bevorzugte wäßrige Fettalkoholdispersionen, die in Mengen von 5 bis 40 Gew.-%, vorzugsweise 15 bis 35 Gew.-% Fettalkohole, gegebenenfalls in Mischung mit natürlichen und/oder synthetischen Wachsen, aliphatische C₁₄-C₂₈-Fettsäuren und/oder Alkalimetall- oder Aminseifen von aliphatischen C₁₄-C₂₈-Fettsäuren,
0,5 bis 5 Gew.-% anionische grenzflächenaktive Verbindungen,
0,5 bis 5 Gew.-% nichtionische grenzflächenaktive Verbindungen,
0 bis 20 % übliche Zusatzstoffe und
der zu 100 Gew. -% fehlende Rest Wasser enthalten.

Übliche Zusatzstoffe sind Schutzkolloide, andere Stabilisatoren oder flüssige Fettalkohole, Fettsäuren, Fette, Öle und Silikonöle, um die Konsistenz der dispergierten Phase zu verbessern, ohne die Wirkung zu beeinträchtigen.

Die erfindungsgemäßen wäßrigen Fettalkoholdispersionen können auf an sich bekannte Art und Weise hergestellt werden. So ist es möglich, den Fettalkohol in eine Schmelze zu überführen und diese in Wasser einzurühren, wobei durch Abkühlung der Schmelze die Dispersionen oder auch vereinzelt Emulsionen entstehen. Man kann aber auch die Fettalkohole in gegebenenfalls erwärmtes Wasser einrühren und das Wasser auf solche Temperaturen führen, bis eine Schmelze entsteht. Durch Zusatz der oben genannten grenzflächenaktiven Verbindungen erfolgt sodann eine Stabilisierung. Eine Gelbildung, wie sie häufig beim Stand der Technik auftritt, erfolgt hier nicht. Falls besonders feinteilige Dispersionen gewünscht werden, kann dies beispielsweise gemäß der DE-A-30 01 387 bei Abkühlung der Schmelze durch Einsatz eines hochscherenden Dissolvers, gegebenenfalls unter Zusatz von bei Raumtemperatur flüssigen Kohlenwasserstoffen, oder beispielsweise gemäß der EP-B-399 266 durch Homogenisierung der Voremulsion und anschließender Abkühlung erreicht werden.

Die erfindungsgemäßen wäßrigen Fettalkoholdispersionen sind für viele Anwendungszwecke geeignet, da sie gut dosiert werden können und lagerstabil sind. Sie zeigen auch bei höheren Gehalten an natürlichen Fettalkoholen eine sehr gute Lagerstabilität, die auch bei Temperaturen unter 15°C erhalten bleibt. Des weiteren sind sie in der Lage, Schaum zu unterdrücken bzw. zu regulieren. Daher betrifft eine weitere Ausführungsform der vorliegenden Erfindung die Verwendung der oben beschriebenen wäßrigen Fettalkoholdispersionen als Antischaummittel, vorzugsweise in der Papierindustrie, im Abwasserbereich, in der Bauindustrie und bei der Herstellung von Wasserlacken. Besonders bevorzugt werden die wäßrigen Fettalkoholdispersionen in der Papierindustrie in wäßrigen Systemen verwendet, deren Temperaturen mindestens 35°C betragen, beispielsweise in der Sulfitzellstoffkochung und in der Papierbeschichtung. Bei der Papierherstellung können sie bei der Mahlung und Dispergierung von Papierstoffen und Pigmenten verwendet werden. Selbstverständlich ist es aber auch möglich, die beschriebenen wäßrigen Fettalkoholdispersionen überall dort einzusetzen, wo Schäume auftreten bzw. verhindert werden sollen, so in der Nahrungsmittelindustrie, der Bauindustrie, der Stärkeindustrie, im Abwasserbereich, insbesondere in Kläranlagen als auch in Lacken und Farben.

Des weiteren wurde gefunden, daS die erfindungsgemäßen wäßrigen Fettalkoholdispersionen als Additiv für dispersionsgebundene Putze geeignet sind, da sie die Verarbeitungszeit verlängern und somit verbessern. Außerdem wird bei der Verwendung der Fettalkoholdispersionen die Neigung zur Rißbildung der dispersionsgebundenen Putze deutlich verringert, eine Verringerung der Wasseraufnahme und damit eine Steigerung der hydrophobierenden Wirkung der Putze, sind weitere Vorteile. Es ergeben sich auch eine gute Überstreichbarkeit der Putze und gute Wasserdampfdurchlässigkeit, so daß insgesamt sich das Eigenschaftsbild von dispersionsgebundenen Putzen in weitem positiv beeinflussen läßt.

### Beispiele

### A. Fettalkoholdispersionen

1) Es wurde eine wäßrige Fettalkoholdispersion aus 23 Gew.-% eines natürlichen C₁₈ bis C₂₂-Fettalkoholgemisches mit etwa 50 Gew.-% Stearylalkohol, 4 Gew.-% Paraffin (Schmelzpunkt 65 bis 67°C), 1 Gew.-% Natriumlaurylethersulfat ethoxyliert mit 30 Mol Ethylenoxid (EO), 2 Gew.% mit Ethylenglykol ringgeöffnetes C12/14 Alkanepoxid ethoxyliert mit 10 Mol EO (erfindungsgemäße nichtionische grenzflächenaktive Verbindung) und 70 Gew.-% Wasser hergestellt. Als Vergleich wurden analoge Fettalkoholdispersionen hergestellt, jedoch mit aus dem Stand der Technik bekannten verschiedenen nichtionischen grenzflächenaktiven Verbindungen (siehe Tabelle 1).
2) Es wurde eine wäßrige Fettalkoholdispersion aus 30 Gew.% eines natürlichen C₁₆ bis C₁₈-Fettalkoholgemisches mit etwa 60 bis 70 Gew.% Stearylalkohol, 0,25 Gew.% Natriumlaurylethersulfat ethoxyliert mit 30 Mol EO, 0,5 Gew.% mit Ethylenglykol ringgeöffnetes C12/14 Alkanepoxid ethoxyliert mit 10 Mol EO und 69,25 Gew.% Wasser hergestellt. Als Vergleich wurde eine analoge Fettalkoholdispersion hergestellt, jedoch mit 0,5 Gew.% Nonylphenol mit 25 mol% EO anstelle der erfindungsgemäßen nichtionischen grenzflächenaktiven Verbindung.

### B. Anwendung als Antischaummitteldispersion

Die nach Beispiel A.1) hergestellten Fettalkoholdispersionen mit den verschiedenen nichtionischen grenzflächenaktiven Verbindungen wurden in Mengen von 150 µl zu einem Liter 7,5 Gew.-%iger Calciumligninsulfonat-Lösung gegeben, die auf 50°C erwärmt worden war. Die Lösung wurde in einer Glasapparatur laufend mit 1000 UpM gerührt, wobei 600 l Luft pro Stunde eingetragen wurden. Bestimmt wurde die Zeit (in Sekunden), nach der die Lösung zusammen mit entstandenem Schaum ein Volumen von 4,5 l einnahm. Die Antischaummittelwirkung ist umso wirkungsvoller, je größer die notwendige Zeitspanne ist, bis das Gesamtvolumen von 4,5 l erreicht ist. In Tabelle 1 sind die Versuchsergebnisse zusammengefaßt. Des weiteren wurde bei Raumtemperatur die Lagerstabilität untersucht. Dazu wurden die wäßrigen Fettalkoholdispersionen mit den verschiedenen nichtionischen grenzflächenaktiven Verbindungen bei Raumtemperatur gelagert. In Tabelle 1 ist der Zeitraum angegeben, bis zu dem eine Lagerstabilität gewährleistet ist, d. h. bis zu dem keine Vergelungen oder Aufrahmungen auftreten.

### C. Lagerstabilität

Die in A2) beschriebenen Fettalkoholdispersionen wurden unter 15 °C gelagert. Die erfindungsgemäßen Fettalkoholdispersionen waren auch nach 4 Wochen unverändert. Die mit Nonylphenol mit 25 mol% EO versetzten Fettalkoholdispersionen zeigten nach maximal 3 Tagen Vergelungen.

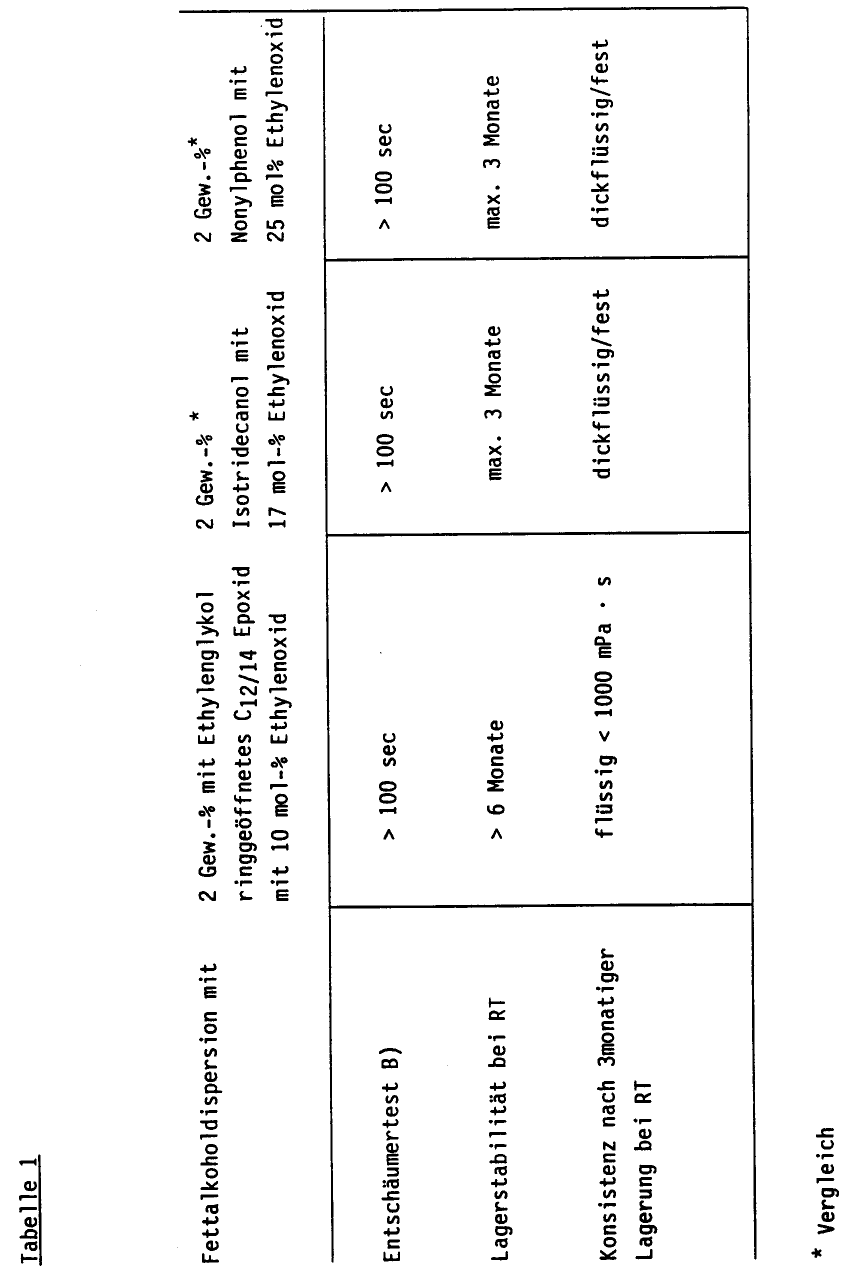

## Patentansprüche

1. Wäßrige Fettalkoholdispersionen enthaltend
- C₁₀-C₂₈ Fettalkohole in der Ölphase sowie
- anionische grenzflächenaktive Verbindungen
- nichtionische grenzflächenaktive Verbindungen und
- Wasser,
dadurch gekennzeichnet, daß die nichtionischen grenzflächenaktive Verbindungen Umsetzungsprodukte von Ethylenoxid mit 1,2-Alkandiolen mit 6 bis 18 C-Atomen sind.

2. Wäßrige Fettalkoholdispersionen nach Anspruch 1, dadurch gekennzeichnet, daß die nichtionischen grenzflächenaktiven Verbindungen solche der allgemeinen Formel I 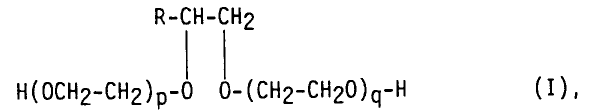 in der
R einen über Kohlenstoff gebundenen gesättigten oder ungesättigten Rest mit 6 bis 18 C-Atomen und
p und q eine Zahl von 0 bis 50 bedeutet, wobei die Summe von p und q im Bereich von 5 bis 50 liegt,
sind.

3. Wäßrige Fettalkoholdispersionen nach Anspruch 1, dadurch gekennzeichnet, daß die Fettalkohole einen Schmelzpunkt über 40°C haben.

4. Wäßrige Fettalkoholdispersionen nach Anspruch 3, dadurch gekennzeichnet, daß die Fettalkohole gesättigt sind und 16 bis 22 C-Atome aufweisen.

5. Wäßrige Fettalkoholdispersionen nach Anspruch 1, dadurch gekennzeichnet, daß die anionischen grenzflächenaktiven Verbindungen Alkoholsulfate, ethoxylierte Alkoholsulfate, Fettalkoholethersulfosuccinate und/oder Fettalkoholetherphosphate sind.

6. Wäßrige Fettalkoholdispersionen nach Anspruch 1, dadurch gekennzeichnet, daß sie 5 bis 40 Gew.-%, vorzugsweise 15 bis 35 Gew.-%, Fettalkohole enthalten.

7. Wäßrige Fettalkoholdispersionen nach Anspruch 6, dadurch gekennzeichnet, daß sie zusätzlich 0,5 bis 10 Gew.-%, an Stelle der entsprechenden Menge Fettalkohol, natürliche und/oder synthetische Wachse, aliphatische C₁₄₋₂₈-Fettsäuren und/oder Alkalimetall- oder Aminseifen von aliphatischen C₁₄₋₂₈-Fettsäuren enthalten.

8. Wäßrige Fettalkoholdispersionen nach Anspruch 1, dadurch gekennzeichnet, daß sie 0,5 bis 5 Gew.-% der nichtionischen grenzflächenaktiven Verbindungen enthalten.

9. Wäßrige Fettalkoholdispersionen nach Anspruch 1, dadurch gekennzeichnet, daß sie 0,5 bis 5 Gew.-% anionische grenzflächenaktive Verbindungen enthalten.

10. Verwendung von wäßrigen Fettalkoholdispersionen nach Anspruch 1 als Antischaummittel, vorzugsweise in der Papierindustrie, im Abwasserbereich, in der Bauindustrie und bei der Herstellung von Wasserlacken.

11. Verwendung von wäßrigen Fettalkoholdispersionen nach Anspruch 1 als Additiv für dispersionsgebundene Putze.

## Claims

1. Aqueous fatty alcohol dispersions containing
- C₁₀₋₂₈ fatty alcohols in the oil phase and
- anionic interfacially active compounds
- nonionic interfacially active compounds and
- water,
characterized in that the nonionic interfacially active compounds are reaction products of ethylene oxide with alkane-1,2-diols containing 6 to 18 carbon atoms.

2. Aqueous fatty alcohol dispersions as claimed in claim 1, characterized in that the nonionic interfacially active compounds corresponding to general formula (I): 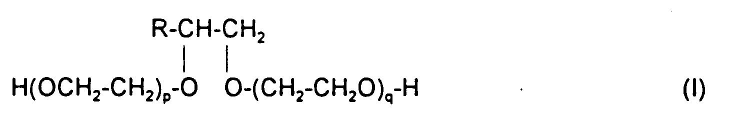 in which R is a saturated or unsaturated radical containing 6 to 18 carbon atoms attached via carbon and p and q are numbers of 0 to 50, the sum of p and q being in the range from 5 to 50.

3. Aqueous fatty alcohol dispersions as claimed in claim 1, characterized in that the fatty alcohols have a melting point above 40°C.

4. Aqueous fatty alcohol dispersions as claimed in claim 3, characterized in that the fatty alcohols are saturated and contain 16 to 22 carbon atoms.

5. Aqueous fatty alcohol dispersions as claimed in claim 1, characterized in that the anionic interfacially active compounds are alcohol sulfates ethoxylated alcohol sulfates, fatty alcohol ether sulfosuccinates and/or fatty alcohol ether phosphates.

6. Aqueous fatty alcohol dispersions as claimed in claim 1, characterized in that they contain 5 to 40% by weight and preferably 15 to 35% by weight of fatty alcohols.

7. Aqueous fatty alcohol dispersions as claimed in claim 6, characterized in that they contain additional 0.5 to 10% by weight of natural and/or synthetic waxes, aliphatic C₁₄₋₂₈ fatty acids and/or alkali metal or amine soaps of aliphatic C₁₄₋₂₈ fatty acids instead of the corresponding quantity of fatty alcohol.

8. Aqueous fatty alcohol dispersions as claimed in claim 1, characterized in that they contain 0.5 to 5% by weight of the nonionic interfacially active compounds.

9. Aqueous fatty alcohol dispersions as claimed in claim 1, characterized in that they contain 0.5 to 5% by weight of anionic interfacially active compounds.

10. The use of the aqueous fatty alcohol dispersions claimed in claim 1 as antifoam agents, preferably in the paper industry, in the wastewater sector, in the building industry and in the production of waterborne paints.

11. The use of the aqueous fatty alcohol dispersions claimed in claim 1 as an additive for dispersion-bonded plasters.

## Revendications

1. Dispersions aqueuses d'alcool gras contenant :
- des alcools gras en C₁₀ à C₂₈ dans la phase huileuse,
- des composés tensioactifs anioniques
- des composés tensioactifs non ioniques, et
- de l'eau,
caractérisées en ce que
les composés tensioactifs non ioniques sont des produits de réaction de l'oxyde d'éthylène avec des 1,2-alcanediols ayant de 6 à 18 atomes de carbone.

2. Dispersions aqueuses d'alcools gras selon la revendication 1,
caractérisées en ce que
les composés tensioactifs non ioniques sont ceux de formule générale (I) 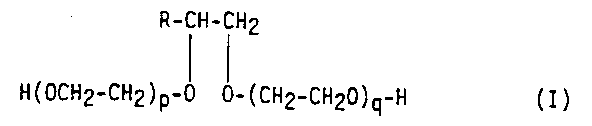 dans laquelle,
R est un radical saturé ou non saturé lié par l'intermédiaire de carbone, ayant de 6 à 18 atomes de carbone et,
p et q signifient un nombre allant de 0 à 50 pour lesquels la somme de p et de q se situe dans la zone allant de 5 à 50.

3. Dispersions aqueuses d'alcools gras selon la revendication 1,
caractérisées en ce que
les alcools gras ont un point de fusion au-dessus de 40°C.

4. Dispersions aqueuses d'alcools gras selon la revendication 3,
caractérisées en ce que
les alcools gras sont saturés et possèdent de 16 à 22 atomes de carbone.

5. Dispersions aqueuses d'alcools gras selon la revendication 1,
caractérisées en ce que
les composés tensioactifs anioniques sont des alcoolsulfates, des alcoolsulfates éthoxylés, des sulfosuccinates d'éthers d'alcool gras, et/ou des phosphates d'éthers d'alcool gras.

6. Dispersions aqueuses d'alcools gras selon la revendication 1,
caractérisées en ce qu'
elles renferment de 5 à 40 % en poids, de préférence de 15 à 35 % en poids, d'alcools gras.

7. Dispersions aqueuses d'alcools gras selon la revendication 6,
caractérisées en ce qu'
elles contiennent en supplément de 0,5 à 10 % en poids à la place de la quantité correspondante d'alcool gras, de cires naturelles et/ou synthétiques, d'acides gras aliphatiques en C₁₄ à C₂₈ et/ou de savons de métaux alcalins ou d'amines d'acides gras en C₁₄ à C₂₈ aliphatiques.

8. Dispersions aqueuses d'alcools gras selon la revendication 1,
caractérisées en ce qu'
elles contiennent de 0,5 à 5 % en poids de composés tensioactifs non ioniques.

9. Dispersions aqueuses d'alcools gras selon la revendication 1,
caractérisées en ce qu'
elles contiennent de 0,5 à 5 % en poids de composants tensioactifs anioniques.

10. Utilisation des dispersions aqueuses d'alcools gras selon la revendication 1, comme agent anti-mousse, de préférence dans l'industrie du papier, dans le domaine des eaux usées, dans l'industrie de la construction et pour la production de laques à l'eau.

11. Utilisation des dispersions aqueuses d'alcools gras selon la revendication 1, comme additifs pour des enduits liés à dispersion.
